(19) Europäisches Patentamt
European Patent Office
Office européen des brevets

(11) EP 3 490 541 B1

(12) EUROPEAN PATENT SPECIFICATION

(45) Date of publication and mention of the grant of the patent:
**09.07.2025 Bulletin 2025/28**

(21) Application number: **17749568.6**

(22) Date of filing: **26.07.2017**

(51) International Patent Classification (IPC):
***A61K 9/70*** (2006.01) ***A61K 31/13*** (2006.01)
***A61K 31/445*** (2006.01) ***A61K 31/137*** (2006.01)
***A61K 31/18*** (2006.01) ***A61K 31/27*** (2006.01)
***A61P 25/00*** (2006.01) ***A61P 25/28*** (2006.01)
***A61P 25/16*** (2006.01) ***A61P 25/04*** (2006.01)
***A61P 25/24*** (2006.01) ***A61P 25/22*** (2006.01)
***A61P 25/36*** (2006.01) ***A61P 35/00*** (2006.01)
***A61P 13/00*** (2006.01) ***A61P 13/02*** (2006.01)
***A61P 25/02*** (2006.01) ***A61P 13/08*** (2006.01)
***A61P 25/14*** (2006.01) ***A61P 25/26*** (2006.01)

(52) Cooperative Patent Classification (CPC):
**A61K 9/7061; A61K 9/7084; A61K 9/7092; A61K 31/13; A61K 31/137; A61K 31/18; A61K 31/27; A61K 31/445; A61P 13/00; A61P 13/02; A61P 13/08; A61P 25/00; A61P 25/02; A61P 25/04; A61P 25/14;** (Cont.)

(86) International application number:
**PCT/US2017/044048**

(87) International publication number:
**WO 2018/022815 (01.02.2018 Gazette 2018/05)**

(54) **TRANSDERMAL FORMULATION AND DELIVERY METHOD OF LOW SOLUBILITY OR UNSTABLE UNIONIZED NEUTRAL DRUGS BYIN SITU**

TRANSDERMALE FORMULIERUNG UND ABGABEVERFAHREN FÜR SCHLECHT LÖSLICHE ODER INSTABILE NICHTIONISIERTE NEUTRALE ARZNEIMITTEL IN SITU

FORMULATION TRANSDERMIQUE ET PROCÉDÉ D'ADMINISTRATION D'UN MÉDICAMENT DE FORME NEUTRE NON IONISÉ DE FAIBLE SOLUBILITÉ OU INSTABLE PAR CONVERSION SEL DE MÉDICAMENTSUR PLACE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**27.07.2016 US 201662367542 P**
**27.07.2016 US 201662367502 P**
**16.11.2016 US 201662423133 P**
**10.01.2017 US 201762444745 P**
**10.01.2017 US 201762444763 P**
**10.02.2017 US 201762457794 P**
**10.05.2017 US 201762504408 P**
**10.05.2017 US 201762504391 P**

(43) Date of publication of application:
**05.06.2019 Bulletin 2019/23**

(73) Proprietor: **Corium, LLC**
**Boston, MA 02210 (US)**

(72) Inventors:
- **LEE, Eun Soo**
**Redwood City**
**California 94061 (US)**
- **JAIN, Amit K.**
**Milpitas**
**California 95035 (US)**
- **SINGH, Parminder**
**Union City**
**California 94587 (US)**
- **SAGI, Appala**
**Mountain View**
**California 94040 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(74) Representative: **D Young & Co LLP**
**3 Noble Street**
**London EC2V 7BQ (GB)**

(56) References cited:

| | |
|---|---|
| **EP-A1- 1 437 130** | **EP-A1- 2 016 941** |
| **EP-A1- 2 514 415** | **EP-A1- 2 818 161** |
| **WO-A1-03/055471** | **WO-A1-03/055471** |
| **WO-A1-2012/089256** | **WO-A1-2012/089256** |
| **WO-A1-2015/053878** | **WO-A1-2017/117554** |
| **CN-A- 1 174 031** | **CN-A- 1 174 031** |
| **CN-A- 105 693 556** | **CN-B- 1 895 242** |
| **CN-B- 1 895 242** | **JP-A- 2015 151 370** |
| **US-A- 5 296 512** | **US-A- 5 296 512** |
| **US-A- 5 730 999** | **US-A- 5 730 999** |
| **US-A1- 2004 018 241** | **US-A1- 2004 018 241** |
| **US-A1- 2008 131 490** | **US-A1- 2008 138 388** |
| **US-A1- 2008 138 388** | **US-A1- 2011 059 141** |
| **US-A1- 2011 313 372** | **US-A1- 2012 245 537** |
| **US-A1- 2014 322 284** | |

- **THE TAMILNADU: "FORMULATION AND EVALUATION OF MATRIX TYPE TRANSDERMAL PATCHES OF BENAZEPRIL HYDROCHLORIDE", DISSERTATION SUBMITTED TO IN PARTIAL FULFILLMENT OF THE REQUIREMENT FOR THE AWARD OF THE DEGREE OF MASTER OF PHARMACY IN PHARMACEUTICS, 1 May 2012 (2012-05-01), XP055688528, Retrieved from the Internet <URL:http://repository-tnmgrmu.ac.in/1716/1/Revathi%20R.pdf> [retrieved on 20200422]**
- **M R SHIVALINGAM ET AL: "Formulation and evaluation of Diclofenac potassium transdermal patches for enhanced therapeutic efficacy", INDIAN JOURNAL OF RESEARCH IN PHARMACY AND BIOTECHNOLOGY, 1 May 2014 (2014-05-01), pages 1152 - 1157, XP055688534, ISSN: 2321-5674**


- **THE TAMILNADU: "FORMULATION AND EVALUATION OF MATRIX TYPE TRANSDERMAL PATCHES OF BENAZEPRIL HYDROCHLORIDE", DISSERTATION SUBMITTED TO IN PARTIAL FULFILLMENT OF THE REQUIREMENT FOR THE AWARD OF THE DEGREE OF MASTER OF PHARMACY IN PHARMACEUTICS, 1 May 2012 (2012-05-01), XP055688528, Retrieved from the Internet <URL:http://repository-tnmgrmu.ac.in/1716/1/Revathi%20R.pdf> [retrieved on 20200422]**
- **M R SHIVALINGAM ET AL: "Formulation and evaluation of Diclofenac potassium transdermal patches for enhanced therapeutic efficacy", INDIAN JOURNAL OF RESEARCH IN PHARMACY AND BIOTECHNOLOGY, 1 May 2014 (2014-05-01), pages 1152 - 1157, XP055688534, ISSN: 2321-5674**


Remarks:
The file contains technical information submitted after the application was filed and not included in this specification

(52) Cooperative Patent Classification (CPC): (Cont.)
**A61P 25/16; A61P 25/22; A61P 25/24; A61P 25/26; A61P 25/28; A61P 25/36; A61P 35/00**

## Description

### TECHNICAL FIELD

The subject matter described herein relates to compositions, devices, and uses of same in transdermal administration of active agents provided in their salt form instead of neutral form, specifically transdermal administration of donepezil.

### BACKGROUND

Active agents for transdermal delivery are typically provided in their neutral form because the neutral form is typically much more skin permeable than a corresponding salt form. In traditional transdermal formulations, a neutral form of an active agent is solubilized in a drug reservoir (also referred to sometimes as an adhesive matrix), and the active agent diffuses from the drug reservoir and into the skin. Transdermal patches, therefore, typically contain as much active agent dissolved in the drug reservoir as the agent's solubility in the components of the 23-30 allows, often with solubilizers to enhance its solubility. Alternatively, neutral, solid particles of active agent are sometimes dispersed in the drug reservoir, so long as the particles' dissolution rate is such that a constant supply of dissolved active agent is provided.

For many active agents, however, a neutral form is difficult to solubilize, formulate, and/or administer to a patient or subject. When a drug has a low solubility as a unionized neutral form in a drug reservoir, it is difficult to incorporate sufficient amount as a solubilized form to deliver at a therapeutic level for multiple days. Moreover, a dissolved active agent may recrystallize as large crystals during the process of solvation, coating, and drying. Further, many active agents are less stable in neutral form than in salt form.

There is a need in the art for improved compositions, devices, patches, systems, and uses for transdermal delivery of active agents that address these shortcomings.

EP 2514415 A1 relates to transdermally absorbed preparations of anti-dementia drugs. US 2011/059141 A1 relates to percutaneous absorption preparations of anti-dementia drugs. US 2011/313372 A1 relates to transdermal therapeutic systems which include, as active ingredient, the NMDA receptor antagonist memantine or one of its physiologically compatible salts. US 2012/245537 A1 relates to a transdermal therapeutic system having a stabilized membrane. WO 2015/053878 A1 relates to topical sphingosine-1-phosphate receptor agonist active agent formulations. US 2014/322284 A1 relates to a composition for making a patch for the transdermal delivery of tamsulosin. CN 105693556 A relates to a transformation method for rivastigmine tartrate and a patch prepared from a product of rivastigmine tartrate. EP 2818161 A1 relates to a method for producing a supersaturated ropinirole patch. US2008131490 discloses a transdermal patch comprising donepezil hydrochloride, sodium hydroxide and a stabilizer solubilized in a pressure-sensitive adhesive layer. EP1437130A1 discloses a percutaneous absorption preparation for treating dementia comprising an adhesive preparation containing donepezil hydrochloride dispersed therein. EP2016941A1 discloses a transdermal preparation which comprises crystalline donepezil having type-B crystal polymorphism.

### BRIEF SUMMARY

The claimed invention is defined by the appended claims.

In one aspect, a composition for transdermal delivery is provided. The composition comprises a drug reservoir comprising micronized particles of an amine salt form of an active agent dispersed in the drug reservoir, a proton accepting entity, and a salt form solubilizer.

The active agent is donepezil. The proton accepting entity is sodium bicarbonate. The salt form solubilizer is selected from the group consisting of water, alcohols, glycerol, propylene glycol, ethylene glycol, dimethyl sulfoxide, and N-methylpyrrolidone.

In one embodiment, the drug reservoir comprises between about 1-70 % w/w of the active agent.

In another embodiment, the drug reservoir comprises between about 0.5-35% w/w of the proton accepting entity.

In one embodiment, the drug reservoir comprises up to 15% w/w of the salt form solubilizer.

In one embodiment, the composition can further comprise a neutral form solubilizer selected from the group consisting of a fatty acid ester, a dicarboxylic acid ester, a glycerol ester, a lactate, a fatty alcohol, sorbitan monolaurate, sorbitan monooleate, lauryl lactate, propylene glycol monolaurate, dimethyl succinate, lauryl alcohol, and oleyl alcohol.

In another embodiment, the drug reservoir comprises up to 15% w/w of the neutral form solubilizer.

In still another embodiment, the composition can comprises a plasticizer selected from the group consisting of a dicarboxylic acid ester, an adipate, a sebacate, a maleate, a tricarboxylic ester, triethyl citrate, tributyl citrate, a glycerol esters, and triacetin.

In yet another embodiment, the drug reservoir comprises up to 20% w/w of the plasticizer.

In another embodiment, the composition comprises an additive selected from the group consisting of crospovidone and colloidal silicone dioxide.

In another embodiment, the drug reservoir comprises up to 25% w/w of additive.

In another embodiment, the drug reservoir comprises an adhesive agent selected from the group consisting of an acrylate, polyisobutylene, silicone adhesive, and styrene block copolymer based adhesive.

In one embodiment, the drug reservoir comprises up to 65% w/w of the adhesive agent.

In another aspect, a transdermal patch comprising a drug reservoir layer as described herein, a backing layer, and a contact adhesive layer is provided.

In one embodiment, the backing layer is an occlusive polymer film.

In one embodiment, the contact adhesive layer comprises an adhesive selected from the group consisting of an acrylate, polyisobutylene, silicone adhesive, and styrene block copolymer based adhesive.

In one embodiment, the transdermal patch further comprises a nonwoven tie layer between the drug reservoir and the contact adhesive layer.

In one embodiment, the transdermal patch further comprises a rate-controlling membrane between the drug reservoir and the contact adhesive layer.

In one embodiment, the transdermal patch comprises at least two drug reservoir layers.

In another embodiment, the at least two drug reservoir layers are separated by a nonwoven tie layer.

## BRIEF DESCRIPTION OF THE FIGURES

FIGS. 1-3 are illustrations of exemplary embodiments of transdermal patch configurations.

FIG. 4 is a graph of average skin flux for donepezil transdermal delivery devices, in $\mu g/cm^2 \cdot hr$, *in vitro* as a function of time, in hours, in an *in vitro* skin permeation test for devices having formulations according to Example 1 (squares), Example 2 (triangles), and Example 7 (circles).

FIG. 5 is a graph of average skin flux for memantine transdermal delivery devices, in $\mu g/cm^2 \cdot hr$, *in vitro* as a function of time, in hours, in an *in vitro* skin permeation test for devices having a formulation according to Example 4.

## DETAILED DESCRIPTION

### I.Definitions

Various aspects now will be described more fully hereinafter. Such aspects may, however, be embodied in many different forms and should not be construed as limited to the embodiments set forth herein; rather, these embodiments are provided so that this disclosure will be thorough and complete, and will fully convey its scope to those skilled in the art.

Where a range of values is provided, it is intended that each intervening value between the upper and lower limit of that range and any other stated or intervening value in that stated range is encompassed within the disclosure. For example, if a range of 1 $\mu$m to 8 $\mu$m is stated, it is intended that 2 $\mu$m, 3 $\mu$m, 4 $\mu$m, 5 $\mu$m, 6 $\mu$m, and 7 $\mu$m are also explicitly disclosed, as well as the range of values greater than or equal to 1 $\mu$m and the range of values less than or equal to 8 $\mu$m.

The singular forms "a," "an," and "the" include plural referents unless the context clearly dictates otherwise. Thus, for example, reference to a "polymer" includes a single polymer as well as two or more of the same or different polymers, reference to a "solvent" includes a single solvent as well as two or more of the same or different solvents.

The use of terms of order or importance, including "first" and "second," is to distinguish and identify individual elements and does not denote or imply a particular order or importance unless clearly indicated by context.

The term "active agent" as used herein refers to a chemical material or compound suitable for topical or transdermal administration and that induces a desired effect. The terms include agents that are therapeutically effective, prophylactically effective, and cosmetically effective agents. The terms "active agent," "drug," and "therapeutic agent" are used interchangeably herein. In the present invention, the active agent is donepezil.

An "adhesive matrix" as described herein includes matrices made in one piece, for example, matrices made via solvent casting or extrusion as well as matrices formed in two or more portions that are then pressed or joined together.

The term "skin" as used herein refers to skin or mucosal tissue, including the interior surface of body cavities that have a mucosal lining. The term "skin" should be interpreted as including "mucosal tissue" and vice versa.

The term "therapeutically effective amount" as used herein refers to the amount of an active agent that is nontoxic but sufficient to provide the desired therapeutic effect. The amount that is "effective" will vary from subject to subject, depending on the age and general condition of the individual, the particular active agent or agents, as known to those skilled in the art.

The terms "transdermal" or "transdermal delivery" as used herein refer to administration of an active agent to a body surface of an individual so that the agent passes through the body surface (*e.g.*, through the skin) and into the

individual's blood stream. The term "transdermal" is intended to include transmucosal administration, *i.e.,* administration of a drug to the mucosal (*e.g.,* sublingual, buccal, vaginal, rectal, *etc.*) surface of an individual so that the agent passes through the mucosal tissue and into the individual's blood stream.

## II. Compositions/Devices

Compositions and/or devices are provided for transdermal administration of active agents. Compositions may be used in devices, patches, and/or systems for transdermal delivery of one or more active agents. Compositions described herein are contemplated for use in transdermal delivery systems, devices, patches, and/or uses as described herein.

Active agents for transdermal delivery are typically provided in their neutral form because the neutral form is typically much more skin permeable than a corresponding salt form. Many active agents, however, are difficult to solubilize in a sufficient amount in a neutral form, difficult to administer in a neutral form at a steady rate for multiple days, and/or less stable in a neutral form than a salt form. As such, the present invention encompasses the recognition that certain active agents are better suited for administration in their salt forms.

In general, compositions described herein provide an active agent in its salt form and at least one proton donating or proton accepting entity. The proton donating or proton accepting entity promotes conversion of the active agent from a salt to its neutral form to improve skin permeability of the active agent. In some embodiments, compositions contain one or more additional ingredients (for example, solubilizers, plasticizers, matrix modifying additives, and/or adhesives). In some embodiments, active agent compositions described herein can be provided in the form of a transdermal drug delivery device, such as a patch.

### A. Compositions for Transdermal Delivery of Active Agents

In a first aspect, compositions comprising: a drug reservoir comprising micronized particles of an amine salt form of an active agent dispersed in the drug reservoir, a proton accepting entity, and a salt form solubilizer are provided. The active agent is donepezil. The proton accepting entity is sodium bicarbonate. The salt form solubilizer is selected from the group consisting of water, alcohols, glycerol, propylene glycol, ethylene glycol, dimethyl sulfoxide, and N-methylpyrrolidone. In general, the salt form of the provided active agent donepezil will react with the provided proton accepting entity sodium bicarbonate in order to generate a neutral form of the active agent that is more skin permeable than the salt form. In some embodiments, the composition may include one or more additional ingredients that cause the neutral form of the active agent to be generated at a specified and/or desired rate. Such compositions can provide, in some embodiments, a relatively constant activity of an active agent.

The compositions may further include one or more of the following: one neutral form solubilizer (total 0 - 50% w/w), one plasticizer for a proton accepting entity and/or proton donating entity (total 0 - 50% w/w), matrix modifying additives (total 0 - 50% w/w), and adhesive polymers (total 0 - 95% w/w).

It will be appreciated that all w/w% or wt% described herein may refer to wet or dry weight of the composition.

In some embodiments, the salt form of at least one active agent is present in the drug reservoir in an amount between about 1-70 wt%, about 1-50 wt%, about 1-35 wt%, about 1-25 wt%, about 2-70 wt%, about 2-50 wt%, about 2-35 wt%, about 5-70 wt%, about 5-50 wt%, about 5-35 wt%, about 5-30 wt%, about 5-25 wt%, about 5-20 wt%, about 5-15 wt%, about 5-10 wt%, about 10-35 wt%, about 10-30 wt%, about 10-25 wt%, about 10-20 wt%, about 10-15 wt%, about 20-35 wt%, about 20-30 wt%, about 20-25 wt%, about 25-35 wt%, about 25-30 wt%, or about 30-35 wt%.

The drug reservoir further comprises a solubilizer that has a limited solubility for the salt form of an active agent (the "salt form solubilizer"). In some embodiments, the micronized particles of a salt form of an active agent will ionize in the salt form solubilizer. In some embodiments, the micronized salt form particles will be maintained in equilibrium with the dissolved, ionized salt form. In some embodiments, the salt form solubilizer has only a limited degree of solubility for the micronized salt particles, such that the equilibrium favors the micronized salt particles over the dissolved, ionized salt form.

In some embodiments, a salt form solubilizer has a solubility for the salt of at least about 0.1 % w/w, at least about 0.2 % w/w, at least about 0.3 % w/w, at least about 0.4 % w/w, at least about 0.5 % w/w, or at least about 1.0 % w/w. In some embodiments, the salt form solubilizer has a solubility for the salt of less than about 30 % w/w or less than about 25% w/w or 20% w/w.

In some embodiments, a salt form solubilizer is a protic solvent (*e.g.,* a solvent that has a hydrogen atom bound to an oxygen (*e.g.,* as in a hydroxyl group) or a nitrogen (*e.g.,* as in an amine group), and/or any solvent that contains labile protons). According to the invention, the salt form solubilizer is selected from the group consisting of water, alcohols (e.g., ethanol, methanol, etc.), glycerol, propylene glycol, ethylene glycol, dimethyl sulfoxide, and N-methylpyrrolidone.

In some embodiments, the drug reservoir comprises about 1-50 wt%, about 1-20 wt%, about 2-50 wt%, about 2-20 wt%, about 5-50 wt%, about 5-20 wt%, about 5-15 wt%, about 5-10 wt%, or about 10-15 wt% of the at least one salt form solubilizer.

In some embodiments, the dissolved, ionized salt form will react with a proton accepting entity (for amine drug

salt), also provided in the matrix. In some embodiments, proton accepting entities are dissolved and intermixed with the drug reservoir. In some embodiments, proton accepting entities are dispersed as fine particles. In some embodiments, proton accepting entities are plasticized.

In some embodiments, a drug reservoir comprises about 0.5-30 wt%, about 1-30 wt%, about 5-30 wt%, about 10-30 wt%, about 15-30 wt%, about 20-30 wt%, about 25-30 wt%, about 0.5-25 wt%, about 1-25 wt%, about 5-25 wt%, about 10-25 wt%, about 15-25 wt%, about 20-25 wt%, about 0.5-20 wt%, about 1-20 wt%, about 5-20 wt%, about 10-20 wt%, about 15-20 wt%, about 0.5-15 wt%, about 1-15 wt%, about 5-15 wt%, about 10-15 wt%, about 0.5-10 wt%, about 1-10 wt%, about 5-10 wt%, about 0.5-5 wt%, or about 1-5 wt% of the proton accepting entity.

Also described herein (although outside the scope of the invention) is a proton accepting entity or proton donating entity which is a polymer. Proton accepting entities may be amine-functional polymers. Exemplary proton accepting polymers include, but are not limited to, amine-functionalized polystyrene miscrosphere, dimethyl aminoethyl methacrylate based acrylates (e.g., EUDRAGIT® EPO or EUDRAGIT® E100), and/or combinations thereof. Proton donating entities may be carboxylic-functional polymers. Exemplary proton donating polymers include, but are not limited to, anionic copolymers based on methacrylic acid and polyacrylic acid (*e.g.*, EUDRAGIT® L100, EUDRAGIT® L100-55, EUDRAGIT® S100), carboxylated polystyrene microsphere, and/or combinations thereof.

The reaction of a salt form of an active agent with a proton accepting polymer or proton donating polymer may generate a skin permeable, neutral form of the active agent and a solid polymer. As the neutral active agent is depleted (by diffusion into the skin) and the polymer becomes separated as a solid phase in the matrix, the reaction continues to move forward, maintaining a relatively steady concentration and/or activity of the neutral form.

In the present disclosure, the proton accepting entity for the amine drug salt is an inorganic salt with mild basicity whose pKa is lower than that of the amine drug salt; specifically sodium bicarbonate which has pKa = 6.4 which is lower than most amine salt drugs. According to the invention, the proton accepting entity is sodium bicarbonate. The reaction products therefore include a skin permeable, neutral form of the active agent, water, and carbon dioxide. As the neutral active agent and $CO_2$ are depleted (by diffusion into the skin and escaped gas, respectively), the reaction continues to move forward, maintaining a relatively steady concentration and/or activity of the neutral form.

In some embodiments, compositions further comprise one or more solubilizers for the neutral form of the active agent (a "neutral form solubilizer"). In some embodiments, the neutral form solubilizer helps ensure that a neutral active agent, once formed, can persist long enough to diffuse into the skin.

In some embodiments, a neutral form solubilizer has a solubility for the neutral form of the active agent of at least about 0.1 % w/w. In some embodiments, the neutral form solubilizer has a solubility for the neutral form of the active agent of less than about 30 % w/w.

In some embodiments, exemplary neutral form solubilizers generally include, but are not limited to, fatty acid esters, lactate esters, dicarboxylic esters, citrate esters, glycerol esters, fatty alcohols, and/or combinations thereof. In some embodiments, exemplary neutral form solubilizers include, but are not limited to, sorbitan monooleate, sorbitan monolaurate (Span® 20), propylene glycol monolaurate, lauryl lactate, dimethyl succinate, triethyl citrate, triacetin, lauryl alcohols, oleyl alcohols, and/or combinations thereof.

In some embodiments, the drug reservoir comprises about 0-40 wt%, about 0-30 wt%, about 0-20 wt%, about 0-15 wt%, about 0-10 wt%, about 0-5 wt%, about 1-40 wt%, about 1-30 wt%, about 1-20 wt%, about 2-40 wt%, about 2-30 wt%, about 2-20 wt%, about 5-20 wt%, about 1-15 wt%, about 2-15 wt%, about 5-15 wt%, about 5-10 wt%, or about 10-15 wt% of at least one neutral form solubilizer.

According to the invention, the active agent is an amine salt drug donepezil. Other amine salt drugs outside the scope of the invention include rivastigmine, memantine, tamsulosin, rotigotine, fentanyl, escitalopram, ropinirole, pramipexole, buprenorphine, fingolimod and lidocaine.

The micronized amine salt form of the active agent is dispersed in a matrix that further comprises at least one salt form solubilizer. The salt form of the active agent ("DHCl") is partially dissolved and ionized, as shown in Equation 1:

$$DHCl \xrightarrow{Ionized} DH^{+} + Cl^{-} \qquad (1)$$

Described herein (although outside the scope of the invention) is where the dissolved, ionized active agent ("$DH^+$") interacts with a proton acceptor amine polymer ("PolymerN") as shown in Equation 2. As the neutral form of the active agent ("D") is depleted by diffusion, the reaction moves toward continuous formation of D.

$$\underset{\text{Dissolved \& skin impermeable amine salt form}}{DH^{+}} + \underset{\text{Solid polymer}}{PolymerN} + Cl^{-} \xrightarrow{Equilibrium\ Constant=K} \underset{\text{Solid polymer}}{PolymerNHCl} + \underset{\text{Dissolved \& skin permeable neutral form}}{\mathrm{D}} \qquad (2)$$

The reaction byproduct ("Polymer NHCl") may be sequestered as solid phase in the matrix. The neutral form of the active agent ("D") may be depleted by diffusion through into the skin.

Because a proton is sequestered from $DH^+$, the dissolved, ionized active agent in the equilibrium reaction (2), the equilibrium constant is expressed as in Equation 3:

$$\boldsymbol{K} = \frac{[\boldsymbol{D}]}{[\boldsymbol{DH^+}]} \qquad (3)$$

The basic inorganic salt (sodium bicarbonate) whose pKa is lower than an amine salt drug can also be used to generate a neutral form of the drug. Sodium bicarbonate has pKa = 6.4. Also described herein is sodium bisulfite as the protein accepting entity for an amine salt drug.

Ionization of sodium bicarbonate is shown in Equation 5, which generates a sodium ion and a bicarbonate ion:

$$NaHCO_3 \xrightarrow{Ionization} Na^+ \quad + \quad HCO_3^- \qquad (5)$$

The salt form of the active agent ("DHCl") is partially dissolved and ionized, as shown in Equation 6:

$$DHCl \xrightarrow{Ionization} DH^+ + \quad Cl^- \qquad (6)$$

8

In some embodiments, a bicarbonate ion accepts a proton from the amine salt drug to form carbonic acid. Carbonic acid decomposes into water and carbon dioxide, which escapes from the patch. As carbon dioxide escapes and the neutral active agent "D" is depleted by diffusion into the skin, the equilibrium of Equation (8) moves toward the right (continuous formation of neutral drug "D").

$$DH^+ + HCO_3^- + \ NaCl \xrightarrow{Equilibrium\ Constant=K} D + H_2\,CO_3 + NaCl -$$

$$\xrightarrow{Further\ reaction} D + H_2O + NaCl \downarrow + CO_2 \uparrow (CO_2\ escapes\ as\ gas) \qquad (8)$$

$$\boldsymbol{K} = \frac{[\boldsymbol{D}]}{[\boldsymbol{DH^+}][\boldsymbol{HCO_3^-}]} \qquad (8)$$

Also described herein (although outside the scope of the invention) is the active agent as an acid salt drug. Such acid salt drugs may have a solubility of at least about 0.1 mg/g, at least about 0.2 mg/g, at least about 0.3 mg/g, at least about 0.4 mg/g, at least about 0.5 mg/g, or at least about 1.0 mg/g in the drug reservoir. The acid salt drugs may have a solubility of less than about 100 mg/g in the drug reservoir. Exemplary acid salt drugs include, but are not limited to sodium alendronate, tresprostinyl sodium, sodium diclofenac, ketoprofen sodium.

A micronized acid salt form of an active agent may be dispersed in a matrix that further comprises at least one salt form solubilizer. The salt form of the active agent ("ANa") is partially dissolved and ionized, as shown in Equation 9:

$$ANa \xrightarrow{Ionized} A^- \quad + \quad Na^+ \qquad (9)$$

The dissolved, ionized active agent ("$A^-$") may interact with a proton donor polymer ("PolymerCOOH") as shown in Equation 10. As the neutral form of the active agent ("AH") is depleted by diffusion, the reaction moves toward continuous formation of AH.

$$A^{-} \quad + \quad PolymerCOOH + Na^{+} \xrightarrow{Equilibrium\ Constant=K} Polymer\ COONa \quad + \quad AH \quad (10)$$

Dissolved & skin impermeable acid salt form — Solid polymer — Solid Polymer — Dissolved & skin permeable neutral form

The reaction byproduct ("Polymer COONa") may be sequestered as solid phase in the matrix. The neutral form of the active agent ("AH") may be depleted by diffusion through into the skin.

Because the reaction byproduct is sequestered as a solid phase in Equation 10, the equilibrium constant is expressed as in Equation 11:

$$\boldsymbol{K} = \frac{[\boldsymbol{AH}]}{[\boldsymbol{A}^{-}]} \qquad (11)$$

In some embodiments, a composition for transdermal delivery of the active agent further comprises one or more plasticizers for the proton accepting or proton donating entity. In some embodiments, salt form solubilizers and/or neutral form solubilizers already present in the composition may also serve as plasticizers for the proton accepting or proton donating entities. In some embodiments, a plasticizer that does not also serve as a salt form and/or neutral form solubilizer is included in a composition. Exemplary plasticizers include, but are not limited to, dicarboxylic acid esters (*e.g.,* adipates, sebacates, maleates, *etc.*), tricarboxylic esters (*e.g.,* triethyl citrate, tributyl citrate, *etc.*), esters of glycerol (*e.g.,* triacetin, *etc.*), and/or combinations thereof.

In some embodiments, an adhesive matrix or drug reservoir comprises about 0-20 wt%, about 0-15 wt%, about 0-10 wt%, about 0-5 wt%, about 5-20 wt%, about 5-15 wt%, about 5-10 wt%, about 10-20 wt%, about 10-15 wt%, or about 15-20 wt% of at least one plasticizer.

In some embodiments, a composition for transdermal delivery of the active agent further comprises at least one additive, also referred to as a matrix modifying additive. In some embodiments, matrix modifying additives modify cohesion and/or diffusivity of described active agent compositions. In some embodiments, matrix modifying additives can absorb moisture and/or water emanating from the skin under occlusion, which improves adhesion to the skin. In some embodiments, matrix modifying additives facilitate homogenization of the drug reservoir. Exemplary matrix modifying additives include, but are not limited to, crospovidone (KOLLIDON® CL-M, *etc.*), cross-linked polyvinylpyrrolidone (PVP), a soluble polyvinylpyrrolidone (PVP), fumed silica, colloidal silicone dioxide, a cellulose derivative (e.g. hydroxypropyl cellulose (HPC), hydroxyethylcellulose (HEC)), a polyacrylamide, a polyacrylic acid, a polyacrylic acid salt, a clay (*e.g.,* kaolin, bentonite, *etc.*), fumed silica, and/or combinations thereof. An exemplary commercial fumed silica product is AEROSIL® 200P, an amorphous, anhydrous colloidal silicon dioxide (Evonik Industries). Another exemplary fumed silica product is Cab-O-Sil® (Cabot Corporation, Boston, Mass.).

In some embodiments, the drug reservoir comprises about 0-25 wt%, about 0-20 wt%, about 0-15 wt%, about 0-10 wt%, about 0-5 wt%, about 5-25 wt%, about 5-20 wt%, about 5-15 wt%, about 5-10 wt%, about 10-25 wt%, about 10-20 wt%, about 10-15 wt%, about 15-25 wt%, about 15-20 wt%, or about 20-25 wt% of at least one matrix modifying additive.

In some embodiments, a composition for transdermal delivery of the active agent further comprises an adhesive or adhesive polymer. Exemplary adhesives and adhesive polymers include, but are not limited to, acrylates, polyisobutylene, silicone adhesive, styrene block copolymer based adhesives, and/or combinations thereof. In some embodiments, proton accepting and/or proton donating polymers are film-formable in the presence of salt form and/or neutral form solubilizers. In such embodiments, compositions may not require a separate adhesive polymer.

In some embodiments, the drug reservoir comprises about 0-65 wt%, about 0-60 wt%, about 0-55 wt%, about 0-50 wt%, about 0-45 wt%, about 0-40 wt%, about 0-35 wt%, about 0-30 wt%, about 0-25 wt%, about 0-20 wt%, about 0-15 wt%, about 0-10 wt%, about 0-5 wt%, 5-65 wt%, about 5-60 wt%, about 5-55 wt%, about 5-50 wt%, about 5-45 wt%, about 5-40 wt%, about 5-35 wt%, about 5-30 wt%, about 5-25 wt%, about 5-20 wt%, about 5-15 wt%, about 5-10 wt%, 10-65 wt%, about 10-60 wt%, about 10-55 wt%, about 10-50 wt%, about 10-45 wt%, about 10-40 wt%, about 10-35 wt%, about 10-30 wt%, about 10-25 wt%, about 10-20 wt%, about 10-15 wt%, 15-65 wt%, about 15-60 wt%, about 15-55 wt%, about 15-50 wt%, about 15-45 wt%, about 15-40 wt%, about 15-35 wt%, about 15-30 wt%, about 15-25 wt%, about 15-20 wt%, 20-65 wt%, about 20-60 wt%, about 20-55 wt%, about 20-50 wt%, about 20-45 wt%, about 20-40 wt%, about 20-35 wt%, about 20-30 wt%, about 20-25 wt%, 25-65 wt%, about 25-60 wt%, about 25-55 wt%, about 25-50 wt%, about 25-45 wt%, about 25-40 wt%, about 25-35 wt%, about 25-30 wt%, 30-65 wt%, about 30-60 wt%, about 30-55 wt%, about 30-50 wt%, about 30-45 wt%, about 30-40 wt%, about 30-35 wt%, 35-65 wt%, about 35-60 wt%, about 35-55 wt%, about 35-50 wt%, about 35-45 wt%, about 35-40 wt%, 40-65 wt%, about 40-60 wt%, about 40-55 wt%, about 40-50 wt%, about 40-45 wt%, 45-65 wt%, about 45-60 wt%, about 45-55 wt%, about 45-50 wt%, 50-65 wt%, about 50-60 wt%, about 50-55 wt%, 55-65 wt%,

about 55-60 wt%, or about 60-65 wt% of at least one adhesive polymer.

The composition may also include other conventional additives such as adhesive agents, antioxidants, crosslinking agents, curing agents, pH regulators, pigments, dyes, refractive particles, conductive species, antimicrobial agents, opacifiers, gelling agents, viscosity modifiers, thickening agents, stabilizing agents, and the like as known in the art. In those embodiments wherein adhesion needs to be reduced or eliminated, conventional detackifying agents may also be used. In some embodiments, agents such as antimicrobial agents are included to prevent spoilage upon storage, *e.g.,* to inhibit growth of microbes such as yeasts and molds. Suitable antimicrobial agents are typically selected from the group consisting of the methyl and propyl esters of p-hydroxybenzoic acid (*e.g.*, methyl and propyl paraben), sodium benzoate, sorbic acid, imidurea, and/or combinations thereof. These additives, and amounts thereof, are selected in such a way that they do not significantly interfere with the desired chemical and physical properties of the adhesive and/or active agent.

Compositions may also contain irritation-mitigating additives to minimize or eliminate the possibility of skin irritation and/or skin damage resulting from the active agent, the proton donating or proton accepting entity, salt form solubilizer, neutral form solubilizer, plasticizer, matrix modifying additive, adhesive and/or other components of the composition. Suitable irritation-mitigating additives include, for example: corticosteroids; $\alpha$-tocopherol; monoamine oxidase inhibitors, particularly phenyl alcohols such as 2-phenyl-1-ethanol; glycerin; salicylic acids and salicylates; ascorbic acids and ascorbates; ionophores such as monensin; amphiphilic amines; ammonium chloride; N-acetylcysteine; cis-urocanic acid; capsaicin; and chloroquine; and/or combinations thereof.

Methods for preparing or manufacturing active agent compositions are also disclosed herein. Exemplary methods are set forth in the Examples section. Methods for preparing active agent compositions generally involve mixing an active agent at 5 - 35% w/w with a proton accepting or proton donating entity at 0.5 - 30% w/w, optionally along with a salt form solubilizer at 0 - 15% w/w, a neutral form solubilizer at 0 - 15% w/w, a matrix modifying additive at 0 - 25% w/w, a plasticizer at 0 - 20% w/w, an adhesive polymer at 0 - 65% w/w, and/or combinations thereof.

B. Transdermal Devices

In certain aspects, the compositions described herein are provided in transdermal devices (e.g., patches). In general, transdermal patches comprise a backing layer, at least one drug reservoir, and a contact adhesive layer. It will be appreciated that the drug reservoir and contact adhesive layer can be in the form of a single layer or matrix comprised of an adhesive and a drug. In some embodiments, transdermal patches further comprise one or more release liners, tie layers, rate-controlling membranes, and/or various combinations of the foregoing.

In some embodiments, transdermal patches comprise one or more of the following components: backing layer, drug reservoir, contact adhesive layer, release liner, tie layer, rate-controlling membrane, and/or various combinations of the foregoing.

Exemplary transdermal patches are shown in FIGS. 1-3. FIG. 1 shows an exemplary transdermal patch **10** comprising a backing layer **12,** multiple drug reservoirs **14, 16** separated by a nonwoven tie layer or a rate controlling membrane **18,** a contact adhesive layer **20,** and a release liner **22.** This particular example presents multiple drug reservoirs separated by a tie layer or a rate controlling membrane **18,** but in some embodiments, multiple adhesive layers may be in direct contact with each other without a tie layer, such as optional tie layer **19.** In such embodiments wherein the transdermal patch comprises multiple drug reservoirs, each drug reservoir may comprise the same or different active agents. In such embodiments wherein the transdermal patch comprises multiple drug reservoirs, each drug reservoir may comprise different concentrations of the same active agent.

FIG. 2 shows an exemplary transdermal patch **30** comprising a backing layer **32,** a drug reservoir **34,** a tie layer or rate-controlling membrane **36,** a contact adhesive layer **38,** and a release liner **39.**

FIG. 3 shows an exemplary transdermal patch **40** comprising a backing layer **42,** a drug reservoir **44,** a contact adhesive layer **46,** and a release liner **48.**

In some embodiments, a backing layer provides a structural element for holding or supporting the adhesive layer. A backing layer may be formed of any suitable material as known in the art. In some embodiments, a backing layer is occlusive. In some embodiments, a backing layer is preferably impermeable or substantially impermeable to moisture. In one exemplary embodiment, the barrier layer has an MVTR (moisture vapor transmission rate) of less than about 50 $g/m^2$-day. In some embodiments, a backing layer is preferably inert and/or does not absorb components of the adhesive layer, including the active agent. In some embodiments, a backing layer preferably prevents release of components of the adhesive layer through the backing layer. A backing layer may be flexible or nonflexible. A backing layer is preferably at least partially flexible such that the backing layer is able to conform at least partially to the shape of the skin where the patch is applied. In some embodiments, a backing layer is flexible such that the backing layer conforms to the shape of the skin where the patch is applied. In some embodiments, a backing layer is sufficiently flexible to maintain contact at the application site with movement, *e.g.,* skin movement. Typically, the material used for a backing layer should permit the device to follow the contours of the skin or other application site and be worn comfortably on areas of skin such as at joints or

other points of flexure, that are normally subjected to mechanical strain with little or no likelihood of the device disengaging from the skin due to differences in the flexibility or resiliency of the skin and the device.

In some embodiments, a backing layer is formed of one or more of a film, non-woven fabric, woven fabric, laminate, and combinations thereof. In some embodiments, the film is a polymer film comprised of one or more polymers. Suitable polymers are known in the art and include, but are not limited to, elastomers, polyesters, polyethylene, polypropylene, polyurethanes, polyether amides, and/or combinations thereof. In some embodiments, a backing layer is formed of one or more of polyethylene terephthalate, various nylons, polypropylene, metalized polyester films, polyvinylidene chloride, aluminum foil, and/or combinations thereof. In some embodiments, a backing layer is a fabric formed of one or more of polyesters such as polyethylene terephthalate, polyurethane, polyvinyl acetate, polyvinylidene chloride, polyethylene, and/or combinations thereof. In one particular, but non-limiting embodiment, the backing layer is formed of a polyester film laminate. Exemplary particular polyester film laminates include, but are not limited to, the polyethylene and/or polyester laminates such as those sold under the names Scotchpak™ #9723, and Scotchpak™ #1012.

In some embodiments, the drug reservoir comprises the salt form of the active ingredient(s) (total 5 - 35% w/w), at least one salt form solubilizer (total up to 20% w/w), at least one neutral form solubilizer (total 0 - 20% w/w), at least one proton accepting entity and optionally at least one proton donating entity (total 0.5 - 30% w/w), optionally at least one plasticizer for a proton accepting entity and/or proton donating entity (total 0 - 20% w/w), matrix modifying additives (total 0 - 25% w/w), and optionally adhesive polymers (total 0 - 65% w/w). In some embodiments, the drug reservoir comprises any of the compositions for transdermal delivery described herein for the invention, e.g., in the Examples and in Section II.A.

In general, a tie layer comprises a nonwoven fabric and/or a rate controlling polymer membrane.

In some embodiments, devices further include one or more fabric or tie layers within or between the drug reservoir layers. It will be appreciated that a tie layer may be included between one, some, or all of the layers. In some embodiments, a tie layer is useful to increase bonding between layers of the device. Tie layers may increase bonding by providing chemical groups for the polymers to bind. In some embodiments, a tie layer is useful as a separation for adhesive matrix layers.

In some embodiments, a tie layer does not affect the rate of release of an active agent from the adhesive layers. In some embodiments, tie layers may comprise nonwoven films that include, but are not limited to, nonwoven polyesters (e.g., REEMAY®), porous polyethylene film (DELNET®), nylon, cotton, and/or combinations thereof.

In some embodiments, tie layers may comprise rate controlling polymer membranes. Exemplary rate controlling polymer membranes include, but are not limited to, microporous polymer films such as CELGARD® 2400 (microporous polypropylene), polyethylenes (e.g., microporous polyethylene), vinyl acetate polymers and copolymers, and/or combinations thereof. In general, a rate controlling polymer membrane allows for a rate-controlled release of the drug from the drug reservoir layer.

In some embodiments, the tie layer comprises a nonwoven fabric and does not comprise a rate controlling polymer membrane. In some embodiments, a tie layer comprises a rate controlling polymer membrane and does not comprise a nonwoven fabric. In some embodiments, a tie layer comprises both a nonwoven fabric and a rate controlling polymer membrane. To give but one example, a nonwoven fabric and a rate controlling polymer membrane may both be used when the tie layer is embedded within the drug reservoir to help improve drug reservoir cohesion (see, *e.g.,* **FIG. 1).**

The device includes at least one adhesive layer. In embodiments, at least one of the adhesive layers is an adhesive matrix comprising one or more active agents as described below. The adhesive layer adheres to a drug reservoir, an adjacent adhesive layer, a tie layer, a release liner, and/or skin at the administration site. In some embodiments, an adhesive layer serves to release the active agent to the skin. In some embodiments, one or more of the drug reservoir adhesive and/or the contact layer adhesive are formed of an adhesive matrix. Exemplary adhesives include, but are not limited to, acrylates, polyisobutylene, a silicone adhesive, a styrene block copolymer based adhesive, and/or combinations thereof.

In some embodiments, the drug reservoir of the delivery system provides an *in vitro* skin flux of an active agent between about 0.5-30 $\mu g/cm^2$-hr for a period of at least about 2 days.

In embodiments, a release liner is at least partially in contact with at least one of the adhesive layers to protect the adhesive layer(s) prior to application. A release liner is typically a disposable layer that is removed prior to application of the device to the treatment site. In some embodiments, a release liner does not absorb components of the adhesive layer(s), including the active agent. In some embodiments, a release liner is impermeable to components of the adhesive layer(s) (including the active agent) and prevents release of components of the adhesive layer(s) through the release liner. In some embodiments, a release liner is formed of one or more of a film, non-woven fabric, woven fabric, laminate, and/or combinations thereof. In some embodiments, a release liner is a silicone-coated polymer film or paper. In some non-limiting embodiments, a release liner is a silicone-coated polyethylene terephthalate (PET) film, a fluorocarbon film, a fluorocarbon coated PET film, and/or combinations thereof.

Transdermal devices and systems (e.g., patches) may be prepared by any suitable methods as known in the art. In some general embodiments, transdermal devices are prepared by coating an appropriate amount of an adhesive polymer composition (with or without an active agent) onto a substrate such as a release liner or a backing layer. In some

embodiments, the adhesive polymer composition is coated onto the release liner. In some embodiments, the adhesive polymer composition is coated onto the substrate or liner to a desired thickness. The thickness and/or size of the device and/or drug reservoir may be determined by one skilled in the art based at least on considerations of wearability and/or required dose. It will be appreciated that the administration site for the device will affect the wearability considerations due to the available size of the administration site and the use of the administration site (*e.g.,* need for flexibility to support movement). In some embodiments, the device and/or drug reservoir has a thickness of between about 25-500 μm. The adhesive polymer composition and substrate are at least partially dried to remove any solvents. A release liner or backing layer is applied to the opposite side of the substrate. Where the substrate is not a release liner or backing layer, the substrate is replaced with the appropriate release liner or substrate. In embodiments that include multiple adhesive polymer layers, a first adhesive polymer composition is applied or coated onto the substrate, a tie layer material is applied to the formulation, and the second adhesive polymer composition is applied to the tie layer material. Adhesive polymer compositions and tie layers are laminated using any suitable methods known in the art. In some embodiments, adhesive layers are coated onto separate substrates or liners and then joined to form the transdermal delivery device. Where the delivery device includes a reservoir adhesive layer and a contact adhesive layer, adhesive polymer compositions may be coated onto the substrate or liner and laminated. It will be appreciated that any or all of the adhesive polymer composition layers may be dried before laminating the layers.

III. Uses

In another aspect, the transdermal compositions, devices, and/or systems described herein for use in treating a disease, condition, and/or disorder by transdermal administration of the at least one active agent are provided, and several non-limiting exemplary embodiments are set forth.

Outside the scope of the invention but described by way of example are compositions which comprise methylphenidate (RITALIN®) as an active agent and are used for treating attention deficit hyperactivity disorder (ADHD), narcolepsy, and/or depression, e.g., through administration of methylphenidate by a transdermal patch. The FDA has approved doses of methylphenidate of 2.5 mg, 5 mg, 10 mg, 15 mg, 18 mg, 20 mg, 27 mg, 30 mg, 36 mg, 40 mg, 50 mg, 54 mg, and 60 mg.

In the present invention, compositions comprising donepezil (ARICEPT®) as an active agent are used for treating Alzheimer's disease, e.g., through administration of donepezil by a transdermal patch. The FDA has approved doses of donepezil of 5 mg, 7 mg, 10 mg, 14 mg, and 23 mg. In some embodiments, compositions described herein allow for administration of doses that correspond to FDA approved doses.

Also described herein but outside the scope of the invention are compositions comprising rivastigmine (Exelon®) as an active agent which are used for treating Alzheimer's disease and/or Parkinson's disease dementia, *e.g.*, through administration of rivastigmine by a transdermal patch. The FDA has approved daily doses of rivastigmine of 1.5 mg, 2.0 mg, 3.0 mg, 4.5 mg, 4.6 mg, 6.0 mg, 9.0 mg, 9.5 mg, and 13.3 mg. Compositions are also described herein comprising memantine as an active agent which are used for treating Alzheimer's disease, obsessive compulsive disorder, anxiety disorder, ADHD, and opioid dependence, *e.g.*, through administration of memantine by a transdermal patch. The FDA has approved doses of memantine of 2 mg, 5 mg, 7 mg, 10 mg, 14 mg, 21 mg, and 28 mg.

Also described herein but outside the scope of the invention are compositions comprising tamsulosin as an active agent which are used for treating benign prostatic hyperplasia and/or acute urinary retention, *e.g.*, through administration of tamsulosin by a transdermal patch. The FDA has approved doses of tamsulosin of 0.4 mg and 0.5 mg.

Transdermal compositions, devices, and/or systems described herein may be designed for long term use and/or continuous administration of the at least one active agent. It will be appreciated that the total dose of the active agent per transdermal device will be determined by the nature of the active agent(s), the size of the device, and/or the loading of the active agent within the drug reservoir. In some embodiments, the application period for the transdermal device is between about 1-10 days, 1-7 days, 1-5 days, 1-2 days, 1-3 days, 1-4 days, 3-10 days, 3-7 days, 3-5 days, 5-10 days, and 5-7 days, inclusive. In some embodiments, the active agent is released from the drug reservoir as a continuous and/or sustained release over the application period.

IV. Examples

The following examples are illustrative in nature and are in no way intended to be limiting. Examples not according to the invention are marked with an asterisk (*).

Unless otherwise specified, the following materials were used in the examples described below: the backing layer was SCOTCHPAK™ 9723; the release liner was a silicone-coated polyester (PET) film; the nonwoven tie layer was REEMAY® 2250; the rate controlling membrane was CELGARD® 2400 microporous polypropylene; and the contact adhesive layer was acrylate, polyisobutene (PIB), and/or silicone adhesive.

EXAMPLE 1*

Donepezil Transdermal Formulation

Preparation of Drug Reservoir

An amount of 9 grams of EUDRAGIT® EPO (a proton accepting polymer) was dissolved in 74.71 grams of ethyl acetate. To the resulting solution, 6 grams of triacetin, 6 grams of glycerin, and 3 grams of SPAN® 20 (sorbitan monolaurate) were added and mixed. To the mixture, 12.6 grams of donepezil hydrochloride powder was dispersed. After addition of 7.8 grams of KOLLIDON® CL-M to the drug dispersed solution, the mixture was homogenized well. To the homogenized drug dispersion, 30.89 grams of DURO-TAK® 387-2287 (solid content 50.5%) was added and mixed well. The wet adhesive formulation was coated on a release liner and dried using a lab coater (Werner Mathis coater) to get a dry coat weight of 10 mg/$cm^2$.

Preparation of Contact Adhesive

An amount of 4.00 grams of triacetin was mixed with 2.00 grams of sorbitan monolaurate and 34.52 grams of ethyl acetate. After addition of 8 grams of KOLLIDON® CL-M, the resulting mixture was homogenized. To the homogenized mixture, 51.48 grams of DURO-TAK® 387-2287 (solid content 50.5%) was added and mixed well. The wet adhesive formulation was coated on a release liner and dried to give a dry coat weight of 5 mg/$cm^2$.

Lamination and Die-cut

CELGARD® 2400 rate controlling membrane or REEMAY® 2250 was laminated onto the adhesive side of the drug reservoir. Contact adhesive was then laminated on top of the CELGARD® 2400 membrane laminated with drug reservoir. The release liner on the drug reservoir side was replaced and laminated with backing film. The final five-layer laminate was die-cut into patches.

*In vitro* skin flux testing

Dermatomed human cadaver skin was obtained from a skin bank and frozen until ready for use. The skin was placed in water at 60 °C for 1-2 mins minute after thawing and the epidermis carefully separated from dermis. The epidermis was either used immediately or wrapped and frozen for later use.

*In vitro* skin flux studies were performed using a Franz type diffusion cell with an active diffusion area of 0.64 $cm^2$. The epidermis was mounted between the donor and receptor compartments of the diffusion cell. The transdermal delivery system was placed over the skin and the two compartments were clamped tight together.

The receptor compartment was filled with 0.01 M phosphate buffer, pH 6.5, containing 0.01% gentamicin. The solution in the receptor compartment was continually stirred using a magnetic stirring bar in the receptor compartment. The temperature was maintained at 32 $\pm$ 0.5 °C. Samples were drawn from the receptor solution at periodic intervals and the receptor solution was replaced with fresh phosphate buffers solution. Drug content in the samples was analyzed using HPLC for donepezil.

The results were calculated in terms of amount of drug diffused through the epidermis per $cm^2$ per unit time. Each data point is an average of four replica of diffusion cells. FIG. 4 shows the *in vitro* skin flux profile of donepezil formulated according to Example 1 (squares).

EXAMPLE 2

Donepezil Transdermal Formulation

Preparation of Drug Reservoir

An amount of 1.20 grams of SPAN® 20 was dissolved in a mixture of 6.00 g of triethyl citrate, and mixed with 1.80 grams of lauryl lactate and 89.69 grams of ethyl acetate. 6.00 grams of glycerin was added and mixed with the SPAN® 20 solution. To the mixture, 9.00 grams of donepezil hydrochloride and 1.82 grams of sodium bicarbonate were dispersed. After addition of 12.00 grams of KOLLIDON® CL-M to the drug dispersed solution, the mixture was homogenized well. To the homogenized drug dispersion, 43.93 grams of DURO-TAK® 387-2287 (solid content 50.5%) was added and mixed well. The wet adhesive formulation was coated on a release liner and dried using a lab coater (Werner Mathis coater) to get a dry coat weight of 12 mg/$cm^2$.

Preparation of Contact Adhesive

An amount of 0.60 grams of SPAN® 20 (sorbitan monolaurate) was dissolved in 3.00 grams of triethyl citrate, and mixed with 0.9 grams of lauryl lactate, 25.45 grams of ethyl acetate, and 1.34 grams of isopropyl alcohol. After addition of 6.00 grams of KOLLIDON® CL-M, the mixture was homogenized. To the homogenized mixture, an amount of 38.61 grams of DURO-TAK® 387-2287 (solid content 50.5%) was added and mixed well. The wet adhesive formulation was coated on a release liner and dried to give a dry coat weight of 5 mg/cm$^2$.

Lamination and Die-cut

CELGARD® 2400 rate controlling membrane or REEMAY® 2250 was laminated on adhesive side of the drug reservoir. Then contact adhesive was laminated on top of the CELGARD® 2400 membrane laminated with drug reservoir. The release liner on the drug reservoir side was replaced and laminated with backing film. The final five-layer laminate was die-cut into patches.

*In vitro* skin flux testing

The *in vitro* skin flux of donepezil from the transdermal system was measured as described in Example 1. FIG. 4 shows the *in vitro* skin flux profile of donepezil (triangles).

EXAMPLE 3*

Tamsulosin Transdermal Formulation

Preparation of Drug Reservoir

An amount of 3.00 grams of EUDRAGIT® EPO was dissolved in 24.51 g of ethyl acetate. To the resulting solution, 2.00 grams of triethyl citrate, 2.00 grams of glycerin, and 1.00 grams of SPAN® 20 (sorbitan monolaurate) were added/mixed. To the mixture, 4.00 grams of tamsulosin hydrochloride powder was dispersed. After addition of 2.4 grams of KOLLIDON® CL-M to the drug dispersed solution, the mixture was homogenized well. To the homogenized drug dispersion, 11.09 grams of DURO-TAK® 387-2287 (solid content 50.5%) was added and well mixed. The wet adhesive formulation was coated on a release liner and dried using a lab coater (Werner Mathis coater) to get a dry coat weight of 10 mg/cm$^2$.

Preparation of Contact Adhesive

An amount of 3.00 grams of triethyl citrate was mixed with 1.50 grams of sorbitan monolaurate (SPAN® 20) and 25.89 grams of ethyl acetate. After addition of 6.00 grams of KOLLIDON® CL-M, the mixture was homogenized. To the homogenized mixture, an amount of 38.61 grams of DURO-TAK 387-2287 (solid content 50.5%) was added and mixed well. The wet adhesive formulation was coated on a release liner and dried to give a dry coat weight of 5 mg/cm$^2$.

Lamination and Die-cut

CELGARD® 2400 rate controlling membrane or REEMAY® 2250 was laminated on adhesive side of the drug reservoir. Then contact adhesive was laminated on top of CELGARD® 2400 membrane laminated with drug reservoir. The release liner on the drug reservoir side was replaced and laminated with backing film. The final five-layer laminate was die-cut into patches.

EXAMPLE 4*

Memantine Transdermal Formulation

Preparation of Drug Reservoir

An amount of 18.00 grams of EUDRAGIT® EPO was dissolved in 14.53 g of ethyl acetate. To the resulting solution, 5.00 grams of triethyl citrate was added and mixed. To the resulting solution, 5.25 grams of memantine hydrochloride powder was added and well dispersed by homogenizing, and then 2.50 grams of glycerin were added and mixed. After addition of 1.75 grams of AEROSIL® 200P to the drug dispersed solution, the dispersion mixture was

homogenized well. To the homogenized dispersion, 2.97 grams of DURO-TAK® 387-2287 (solid content 50.5%) was added and well mixed. The wet adhesive formulation was coated on a release liner and dried using a lab coater (Werner Mathis coater) to get a dry coat weight of 15 mg/cm$^2$.

Preparation of Contact Adhesive

An amount of 3.00 grams of triethyl citrate was mixed with 17.26 grams of ethyl acetate. After addition of 4.00 grams of KOLLIDON® CL-M the mixture was homogenized. To the homogenized mixture, an amount of 25.74 grams of DURO-TAK® 387-2287 (solid content 50.5%) was added and mixed well. The wet adhesive formulation was coated on a release liner and dried to give a dry coat weight of 5 mg/cm$^2$.

Lamination and Die-cut

CELGARD® 2400 rate controlling membrane was laminated to the drug reservoir. Then contact adhesive was laminated to the other side of CELGARD® 2400 membrane laminated with the drug reservoir. The release liner on the drug reservoir was replaced and laminated with backing film. The final five-layer laminate was die-cut into patches.

*In vitro* skin flux testing

The *in vitro* skin flux of memantine from the transdermal system was measured as described in Example 1, where memantine content in the samples drawn from receptor solution were analyzed for memantine using LCMS. FIG. 5 shows the *in vitro* skin flux profile of memantine.

EXAMPLES 5-8

Additional Transdermal Formulations

Additional formulations were prepared according to the ingredients and quantities set forth in Table 1 and the general methods set forth in Example 1. FIG. 4 shows a flux profile of donepezil formulated according to the formulation identified as Example No. 7 in Table 1 below (circles).

TABLE 1

| Example No. --> | | 1* | 2 | 3* | 4* | 5 | 6* | 7* | 8* |
|---|---|---|---|---|---|---|---|---|---|
| | | Composition (w/w %) | | | | | | | |
| Drug reservoir | Donepezil HCl | 21 | 15 | - | - | 15 | 25 | 24 | 25.2 |
| | Tamsulosin HCl | - | - | 20 | - | - | - | - | - |
| | Memantine HCl | | | | 21 | | | | |
| | Triethyl citrate | - | 10 | 10 | 20 | 10 | 10 | - | - |
| | Triacetin | 10 | - | - | - | - | - | 10 | - |
| | Sorbitan monolaurate | 5 | 2 | - | - | 5 | - | 5 | - |
| | Lauryl lactate | - | 3 | - | - | - | 6 | - | 5 |
| | Dimethyl succinate | - | - | - | - | - | - | - | 5 |
| | Glycerin | 10 | 10 | 10 | 10 | 10 | 10 | 10 | - |
| | Dimethylsulfoxide | - | - | - | - | - | - | - | 1 |
| | Eudragit EPO | 15 | - | 15 | 36 | - | 17.7 | 17 | 17.8 |
| | Sodium bicarbonate | - | 3.03 | - | - | 0.75 | - | - | - |
| | Kollidon CL-M | 13 | 15 | 12 | - | 20 | - | | - |
| | Aerosil 200P | | - | - | 7 | - | 7 | 7 | 7 |
| | Duro-Tak 387-2287 | 26 | 36.97 | 28 | 6 | 37.7 | | 27 | - |
| | Duro-Tak 387-2516 | - | - | - | - | - | 31.3 | - | 29 |

(continued)

| Example No. --> | | 1* | 2 | 3* | 4* | 5 | 6* | 7* | 8* |
|---|---|---|---|---|---|---|---|---|---|
| | | Composition (w/w %) | | | | | | | |
| RCM or tie layer | Celgard 2400 | yes | yes | yes | yes | - | yes | yes | yes |
| | Reemay 2250 | - | - | - | - | yes | - | - | - |
| Contact adhesive | Triethyl citrate | - | 10 | 10 | 15 | 10 | 10 | - | - |
| | Triacetin | 10 | - | - | - | - | - | 10 | - |
| | Sorbitan monolaurate | 5 | 2 | 5 | - | 5 | - | 5 | - |
| | Lauryl lactate | - | 3 | - | - | - | 6 | - | 5 |
| | Dimethyl succinate | - | - | - | - | - | - | - | 5 |
| | Dimethyl sulfoxide | - | - | - | - | - | - | - | 1 |
| | Kollidon CL-M | 20 | 20 | 20 | 20 | 20 | 20 | - | - |
| | Aerosil 200P | | - | - | - | - | - | 7 | 7 |
| | Duro-Tak 387-2287 | 65 | 65 | 65 | 65 | 65 | 64 | 78 | - |
| | Duro-Tak 387-2516 | - | - | - | - | - | - | - | 82 |
| 168 hour average flux ($\mu g/cm^2$-hr) | | 4.88 | 5.09 | N/A | 8.16 | 8.16 | 4.61 | 4.58 | 3.25 |

While a number of exemplary aspects and embodiments have been discussed above, those of skill in the art will recognize certain modifications, permutations, additions and sub-combinations thereof. It is therefore intended that the following appended claims are interpreted to include all such modifications, permutations, additions and sub-combinations as are within their scope.

## Claims

**1.** A composition for transdermal delivery, comprising:

a drug reservoir comprising micronized particles of an amine salt form of an active agent dispersed in the drug reservoir, a proton accepting entity, and a salt form solubilizer;
wherein the active agent is donepezil;
wherein the proton accepting entity is sodium bicarbonate; and
wherein the salt form solubilizer is selected from the group consisting of water, alcohols, glycerol, propylene glycol, ethylene glycol, dimethyl sulfoxide, and N-methylpyrrolidone.

**2.** The composition of claim 1, wherein the drug reservoir comprises 1% to 70 % w/w of the active agent.

**3.** The composition of any of the preceding claims, wherein the drug reservoir comprises 0.5% to 35% w/w of the proton accepting entity.

**4.** The composition of any of the preceding claims, wherein the drug reservoir comprises up to 15% w/w of the salt form solubilizer.

**5.** The composition of any of the preceding claims, further comprising a neutral form solubilizer selected from the group consisting of a fatty acid ester, a dicarboxylic acid ester, a glycerol ester, a lactate, a fatty alcohol, sorbitan monolaurate, sorbitan monooleate, lauryl lactate, propylene glycol monolaurate, dimethyl succinate, lauryl alcohol, and oleyl alcohol, preferably wherein the drug reservoir comprises up to 15% w/w of the neutral form solubilizer.

**6.** The composition of any of the preceding claims, further comprising a plasticizer selected from the group consisting of a dicarboxylic acid ester, an adipate, a sebacate, a maleate, a tricarboxylic ester, triethyl citrate, tributyl citrate, a glycerol esters, and triacetin, preferably wherein the drug reservoir comprises up to 20% w/w of the plasticizer.

7. The composition of any of the preceding claims, further comprising an additive selected from the group consisting of crospovidone and colloidal silicone dioxide, preferably wherein the drug reservoir comprises up to 25% w/w of the additive.

8. The composition of any of the preceding claims, wherein the drug reservoir comprises an adhesive agent selected from the group consisting of an acrylate, polyisobutylene, silicone adhesive, and styrene block copolymer based adhesive, preferably wherein drug reservoir comprises up to 65% w/w of the adhesive agent.

9. A transdermal patch comprising a drug reservoir layer comprising a composition of any one of the preceding claims; a backing layer; and a contact adhesive layer.

10. The transdermal patch of claim 9, wherein the backing layer is an occlusive polymer film.

11. The transdermal patch of claim 9 or claim 10, wherein the contact adhesive layer comprises an adhesive selected from the group consisting of an acrylate, polyisobutylene, silicone adhesive, and styrene block copolymer based adhesive.

12. The transdermal patch of any of claims 9-11, further comprising a nonwoven tie layer between the drug reservoir and the contact adhesive layer or a rate-controlling membrane between the drug reservoir layer and the contact adhesive layer.

13. The transdermal patch of any of claims 9-12, wherein the patch comprises at least two drug reservoir layers preferably wherein at least two drug reservoir layers are separated by a nonwoven tie layer.

14. The transdermal patch of claim 13, wherein at least two drug reservoir layers are separated by a rate-controlling membrane.

15. A composition according to any of claims 1-8 or a transdermal patch according to any of claims 9-14 for use in therapy, wherein the active agent of the composition or patch is administered transdermally.

16. A composition according to any of claims 1-8 or a transdermal patch according to any of claims 9-14 for use in the treatment of Alzheimer’s disease.

**Patentansprüche**

1. Zusammensetzung zur transdermalen Zuführung, umfassend:

   ein Arzneistoffreservoir, das mikronisierte Partikel einer Aminsalzform eines in dem Arzneistoffreservoir dispergierten Wirkstoffs, eine protonenakzeptierende Entität und einen Salzform-Lösungsvermittler umfasst;
   wobei es sich bei dem Wirkstoff um Donepezil handelt;
   wobei es sich bei der protonenakzeptierenden Entität um Natriumhydrogencarbonat handelt; und
   wobei der Salzform-Lösungsvermittler aus der Gruppe bestehend aus Wasser, Alkoholen, Glycerin, Propylenglykol, Ethylenglykol, Dimethylsulfoxid und N-Methylpyrrolidon ausgewählt ist.

2. Zusammensetzung nach Anspruch 1, wobei das Arzneistoffreservoir 1 Gew.-% bis 70 Gew.-% des Wirkstoffs umfasst.

3. Zusammensetzung nach einem der vorhergehenden Ansprüche, wobei das Arzneistoffreservoir 0,5 Gew.-% bis 35 Gew.-% der protonenakzeptierenden Entität umfasst.

4. Zusammensetzung nach einem der vorhergehenden Ansprüche, wobei das Arzneistoffreservoir bis zu 15 Gew.- % des Salzform-Lösungsvermittlers umfasst.

5. Zusammensetzung nach einem der vorhergehenden Ansprüche, ferner umfassend einen Lösungsvermittler in neutraler Form, der aus der Gruppe bestehend aus einem Fettsäureester, einem Dicarbonsäureester, einem Glycerinester, einem Lactat, einem Fettalkohol, Sorbitanmonolaurat, Sorbitanmonooleat, Lauryllactat, Propylenglykolmonolaurat, Bernsteinsäuredimethylester, Laurylalkohol und Oleylalkohol ausgewählt ist, vorzugsweise wobei das Arzneistoffreservoir bis zu 15 Gew.-% des neutralen Lösungsvermittlers umfasst.

**6.** Zusammensetzung nach einem der vorhergehenden Ansprüche, ferner umfassend einen Weichmacher, der aus der Gruppe bestehend aus einem Dicarbonsäureester, einem Adipat, einem Sebacat, einem Maleat, einem Tricarbonsäureester, Triethylcitrat, Tributylcitrat, einem Glycerinester und Triacetin ausgewählt ist, vorzugsweise wobei das Arzneistoffreservoir bis zu 20 Gew.-% des Weichmachers umfasst.

**7.** Zusammensetzung nach einem der vorhergehenden Ansprüche, ferner umfassend ein Additiv, das aus der Gruppe bestehend aus Crospovidon und kolloidalem Silikondioxid ausgewählt ist, vorzugsweise wobei das Arzneistoffreservoir bis zu 25 Gew.-% des Additivs umfasst.

**8.** Zusammensetzung nach einem der vorhergehenden Ansprüche, wobei das Arzneistoffreservoir ein Haftmittel umfasst, das aus der Gruppe bestehend aus einem Acrylat- , Polyisobutylen-, Silikonklebstoff und einem Klebstoff auf Styrolblockcopolymer-Basis ausgewählt ist, vorzugsweise wobei das Arzneistoffreservoir bis zu 65 Gew.-% des Haftmittels umfasst.

**9.** Transdermales Pflaster, umfassend eine Arzneistoffreservoirschicht, die eine Zusammensetzung nach einem der vorhergehenden Ansprüche umfasst; eine Trägerschicht; und eine Haftkleberschicht.

**10.** Transdermales Pflaster nach Anspruch 9, wobei es sich bei der Trägerschicht um eine okklusive Polymerfolie handelt.

**11.** Transdermales Pflaster nach Anspruch 9 oder Anspruch 10, wobei die Haftkleberschicht einen Klebstoff umfasst, der aus der Gruppe bestehend aus einem Acrylat-, Polyisobutylen-, Silikonklebstoff und einem Klebstoff auf Styrolblockcopolymerbasis ausgewählt ist.

**12.** Transdermales Pflaster nach einem der Ansprüche 9-11, ferner umfassend eine Vliesbindeschicht zwischen dem Arzneistoffreservoir und der Kontaktkleberschicht oder eine geschwindigkeitsbestimmende Membran zwischen der Arzneistoffreservoirschicht und der Kontaktkleberschicht.

**13.** Transdermales Pflaster nach einem der Ansprüche 9-12, wobei das Pflaster wenigstens zwei Arzneistoffreservoirschichten umfasst, vorzugsweise wobei wenigstens zwei Arzneistoffreservoirschichten durch eine Vliesbindeschicht getrennt sind.

**14.** Transdermales Pflaster nach Anspruch 13, wobei wenigstens zwei Arzneistoffreservoirschichten durch eine geschwindigkeitsbestimmende Membran getrennt sind.

**15.** Zusammensetzung gemäß einem der Ansprüche 1-8 oder transdermales Pflaster gemäß einem der Ansprüche 9-14 zur therapeutischen Verwendung, wobei der Wirkstoff der Zusammensetzung bzw. des Pflasters transdermal verabreicht wird.

**16.** Zusammensetzung gemäß einem der Ansprüche 1-8 oder transdermales Pflaster gemäß einem der Ansprüche 9-14 zur Verwendung bei der Behandlung von Morbus Alzheimer.

## Revendications

**1.** Composition pour administration transdermique, comprenant :

un réservoir de médicament comprenant des particules micronisées d'une forme de sel d'amine d'un agent actif dispersées dans le réservoir de médicament, une entité acceptant les protons et un solubilisant sous forme de sel ;
dans laquelle l'agent actif est le donépézil ;
dans laquelle l'entité acceptant les protons est le bicarbonate de sodium ; et
dans laquelle le solubilisant sous forme de sel est choisi dans le groupe constitué par l'eau, les alcools, le glycérol, le propylène glycol, l'éthylène glycol, le diméthylsulfoxyde et la N-méthylpyrrolidone.

**2.** Composition selon la revendication 1, dans laquelle le réservoir de médicament comprend 1 % à 70 % p/p de l'agent actif.

**3.** Composition selon l'une quelconque des revendications précédentes, dans laquelle le réservoir de médicament

comprend 0,5 % à 35 % p/p de l'entité acceptant les protons.

4. Composition selon l'une quelconque des revendications précédentes, dans laquelle le réservoir de médicament comprend jusqu'à 15 % p/p du solubilisant sous forme de sel.

5. Composition selon l'une quelconque des revendications précédentes, comprenant en outre un solubilisant sous forme neutre choisi dans le groupe constitué par un ester d'acide gras, un ester d'acide dicarboxylique, un ester de glycérol, un lactate, un alcool gras, le monolaurate de sorbitane, le monooléate de sorbitane, le lactate de lauryle, le monolaurate de propylène glycol, le succinate de diméthyle, l'alcool laurylique, et l'alcool oléylique, préférablement dans laquelle le réservoir de médicament comprend jusqu'à 15 % p/p du solubilisant sous forme neutre.

6. Composition selon l'une quelconque des revendications précédentes, comprenant en outre un plastifiant choisi dans le groupe constitué par un ester d'acide dicarboxylique, un adipate, un sébacate, un maléate, un ester tricarboxylique, le citrate de triéthyle, le citrate de tributyle, des esters de glycérol et la triacétine, de préférence dans laquelle le réservoir de médicament comprend jusqu'à 20 % p/p du plastifiant.

7. Composition selon l'une quelconque des revendications précédentes, comprenant en outre un additif choisi dans le groupe constitué par une crospovidone et un dioxyde de silicone colloïdal, de préférence dans laquelle le réservoir de médicament comprend jusqu'à 25 % p/p de l'additif.

8. Composition selon l'une quelconque des revendications précédentes, dans laquelle le réservoir de médicament comprend un agent adhésif choisi dans le groupe constitué par un acrylate, un polyisobutylène, un adhésif de silicone et un adhésif à base de copolymère à blocs de styrène, de préférence dans laquelle le réservoir de médicament comprend jusqu'à 65 % p/p de l'agent adhésif.

9. Patch transdermique comprenant une couche de réservoir de médicament comprenant une composition selon l'une quelconque des revendications précédentes ; une couche de support ; et une couche d'adhésif de contact.

10. Patch transdermique selon la revendication 9, dans lequel la couche de support est un film de polymère occlusif.

11. Patch transdermique selon la revendication 9 ou la revendication 10, dans lequel la couche d'adhésif de contact comprend un adhésif choisi dans le groupe constitué par un acrylate, un polyisobutylène, un adhésif de silicone et un adhésif à base de copolymère à blocs de styrène.

12. Patch transdermique selon l'une quelconque des revendications 9 à 11, comprenant en outre une couche de liaison non tissée entre le réservoir de médicament et la couche d'adhésif de contact ou une membrane de régulation de débit entre la couche de réservoir de médicament et la couche d'adhésif de contact.

13. Patch transdermique selon l'une quelconque des revendications 9 à 12, dans lequel le patch comprend au moins deux couches de réservoir de médicament, de préférence dans lequel au moins deux couches de réservoir de médicament sont séparées par une couche de liaison non tissée.

14. Patch transdermique selon la revendication 13, dans lequel au moins deux couches de réservoir de médicament sont séparées par une membrane régulant le débit.

15. Composition selon l'une quelconque des revendications 1 à 8 ou patch transdermique selon l'une quelconque des revendications 9 à 14 pour une utilisation en thérapie, dans lequel l'agent actif de la composition ou du patch est administré par voie transdermique.

16. Composition selon l'une quelconque des revendications 1 à 8 ou patch transdermique selon l'une quelconque des revendications 9 à 14 pour une utilisation dans le traitement de la maladie d'Alzheimer.

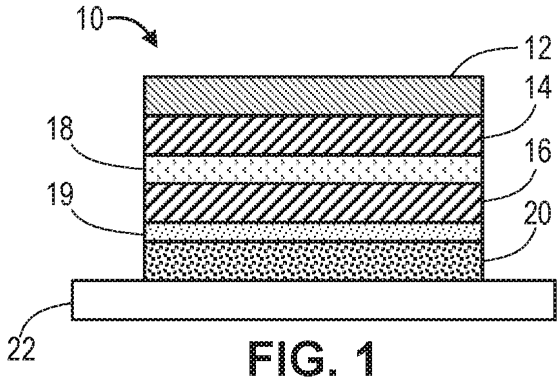


FIG. 1

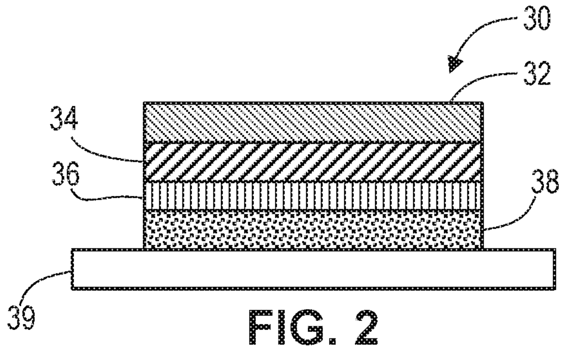


FIG. 2

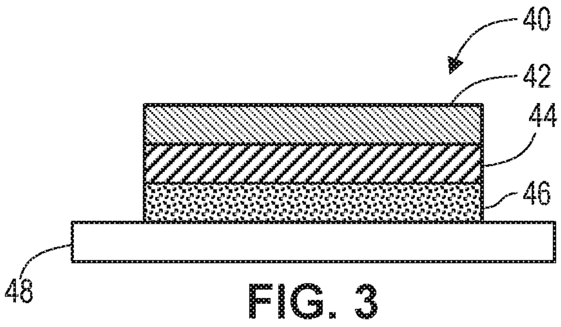


FIG. 3

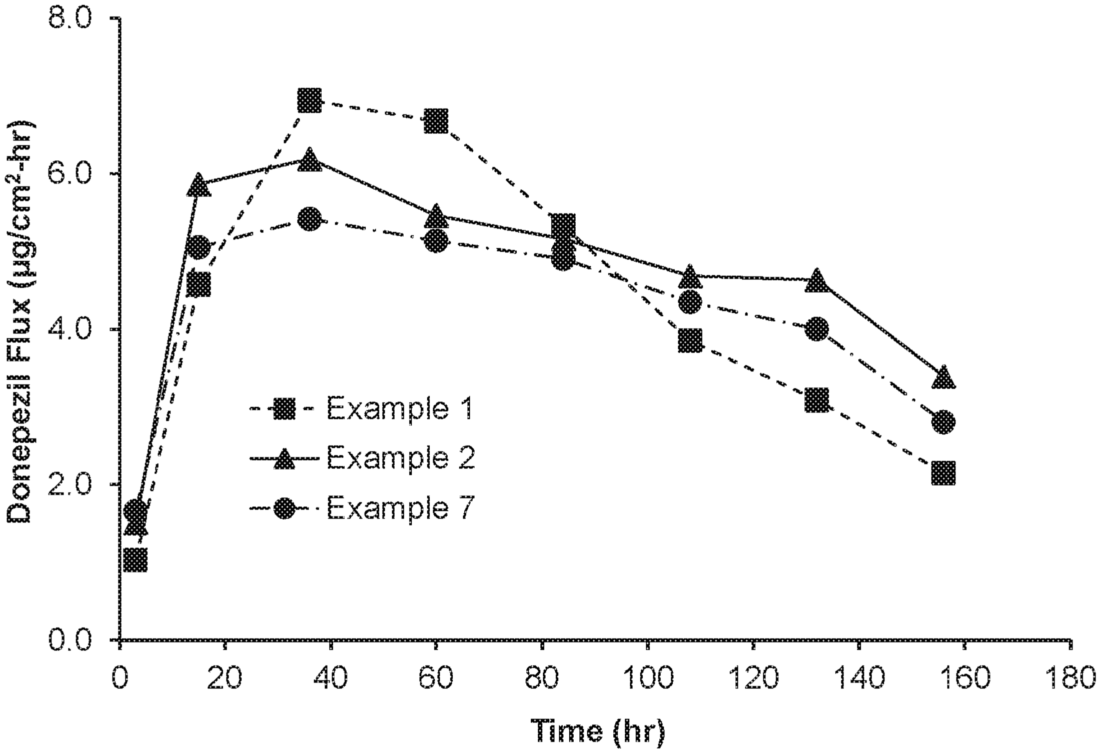
8.0
6.0
4.0
2.0
0.0
Donepezil Flux (µg/cm²-hr)
0
20
40
60
80
100
120
140
160
180
Time (hr)
Example 1
Example 2
Example 7

FIG. 4

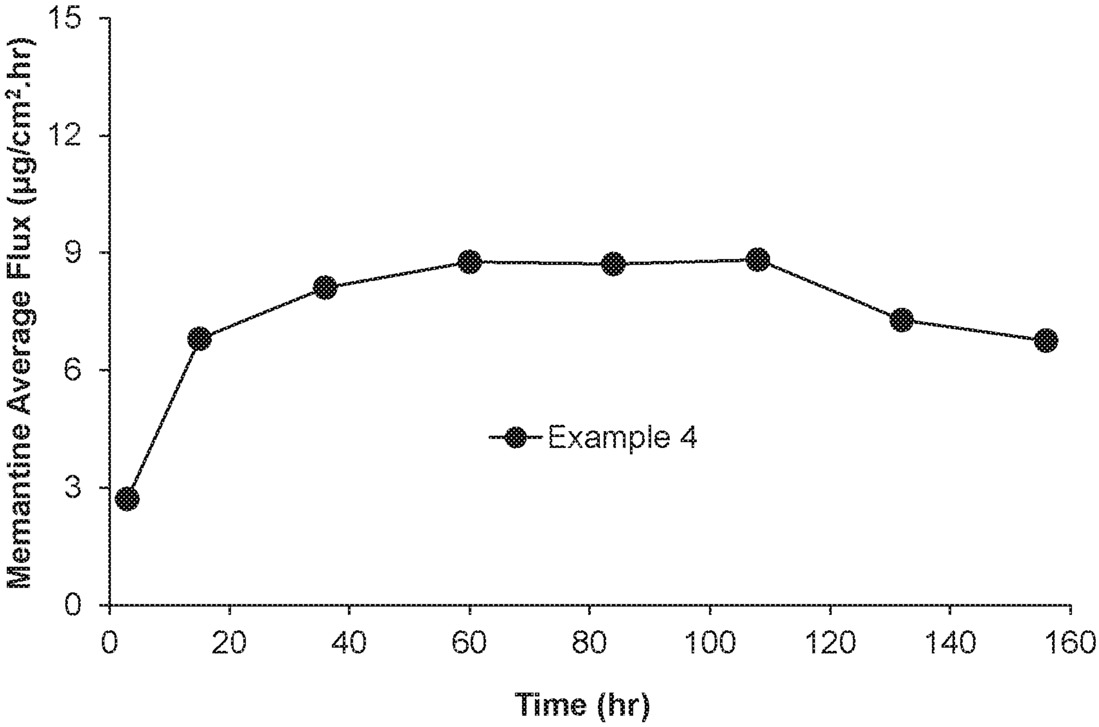

15
12
9
6
3
0
Memantine Average Flux (μg/cm².hr)
0
20
40
60
80
100
120
140
160
Time (hr)
Example 4


FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2514415 A1
- US 2011059141 A1
- US 2011313372 A1
- US 2012245537 A1
- WO 2015053878 A1
- US 2014322284 A1
- CN 105693556 A
- EP 2818161 A1
- US 2008131490 A
- EP 1437130 A1
- EP 2016941 A1